# EUROPEAN PATENT APPLICATION

(11) **EP 1 911 752 A1**
(43) Date of publication of application: **16.04.2008**
(21) Application number: 06020774.3
(22) Date of filing: 03.10.2006
(51) Int. Cl.: C07D 307/02

(54) **Process for the preparation of butyrolactones**

(71) Applicant: Lonza AG, 4052 Basel (CH)
(72) Inventor: The designation of the inventor has not yet been filed

(57) **Abstract**

The present invention relates to a process for the preparation of γ-butyrolactones.

## Description

The present invention relates to a process for the preparation of γ-butyrolactones of formulae 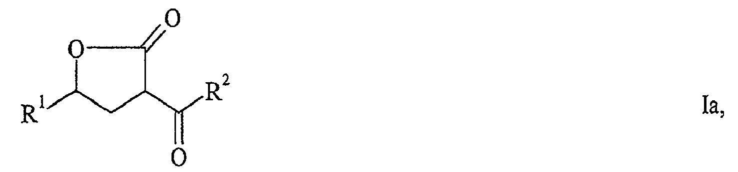 wherein R¹ is hydrogen or C₁₋₁₀-alkyl and R² is selected from the group consisting of C₁₋₁₉ alkyl, aryl and aralkyl, 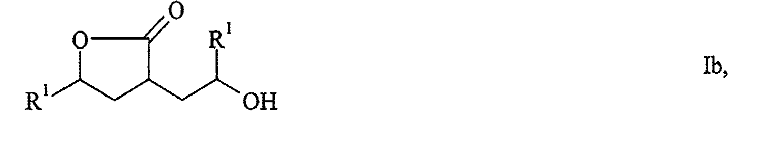 wherein R¹ is as defined above,
and/or 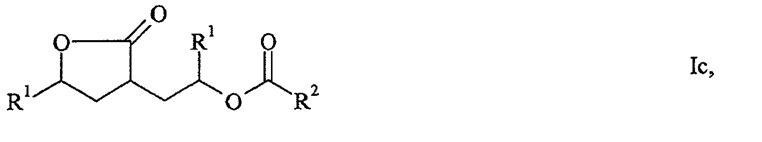 wherein R¹ and R² are as defined above.
3-Acetyl-dihydro-2(3H)-furanone or α-acetylbutyrolactone (ABL) is a fine chemical used as an intermediate in the production of vitamin B1 (M. Eggersdorfer, et al., Vitamins in Ullmann's Encyclopedia of Industrial Chemistry, Ed. M. Bohnet et al., Wiley, New York, 2005, 71), agrochemicals (WO-A-96/16048) and other fine chemicals, such as cyclopropylamine (DE-A-3827846). The worldwide use of ABL totals to greater than 10000 tons.

ABL is prepared either by acetylation of γ-butyrolactone (dihydro-2(3H)-furanone, GBL) according to EP-A-792877 or by the reaction of alkyl acetoacetates with ethylene oxide. Several procedures were described for the latter reaction.

According to US-A-2443827 ethyl acetoacetate and ethylene oxide are reacted with NaOH in a H₂O/EtOH-mixture for 48 h at 0 °C. The base is neutralized with AcOH. Extraction with benzene and fractional distillation furnishes 60% of ABL.

According to JP-A-2000355588 and JP-A-2002173489, methyl acetoacetate and 1.05 eq. of ethylene oxide are reacted with 1.0 eq. of NaOH in MeOH at 25 °C for 13 h. This results in the conversion of 71 % of the methyl acetoacetate and the formation of ABL with a selectivity of 82%. After removal of MeOH, neutralisation with H₂SO₄, toluene extraction and distillation, ABL is isolated in 78% yield based on the consumed methyl acetoacetate.

According to US-A-5183908 and EP-A-0348549 substituted α-acetylbutyrolactones were prepared from C₁₋₂₀ alkyl acetoacetates and epoxides with NaOH in H₂O at 15 to 34°°C. By using higher branched chain alkyl acetoacetates there is no need of a solvent for the extractive workup, as the alcohol produced as a by-product of the reaction is immiscible with H₂O.

According to EP-A-0702998 NaOMe can be recovered from the reaction mixture of ABL production by electrodialysis. However, this workup requires long times (exceeding 20 h).

An alternative reaction was disclosed by Ishido et al. in J. Chem. Soc., Perkin Trans. 1 1977, 521, wherein ethyl acetoacetate is reacted with ethylene carbonate in the presence of catalytic amounts of sodium iodide at 150 - 155 °C for 4 h to furnish ABL with a low yield of 17%.

EP-A-0588224 discloses the reaction of ethyl acetoacetate with 2.0 eq. of ethylene oxide to give 3-(2'-acetoxyethyl)-dihydro-2-(3H)furanone (72% isolated) in the presence of 0.1 eq. of NaOMe in MeOH at 60 °C for 24 h and the formation of ABL as a by-product (3% isolated).

Besides long reaction times, all procedures mentioned above are characterised by the use of stoichiometric amounts of an inorganic base (alkali metal hydroxides, alkali metal alkoxides or ammonium halides) and thus by the production of large amounts of salts. Such a production of waste salts is clearly undesirable in a large scale production of ABL. Therefore, a process using only catalytic amounts of a base or a recyclable base is highly desirable.

Packendorff et al. reported in Bull. Acad Sci. USSR. 1940, 24, 579 the reaction of ethyl acetoacetate with 2.17 eq. of ethylene oxide in the presence of 0.02 eq. of piperidine at RT for 20 d affording (2'-acetoxyethyl)-dihydro-2-(3H)furanone. ABL itself is not mentioned.

A general method which provides improved formation of ABL and a reduction of salt waste would therefore be advantageous. Particularly, methods for performing the reaction in reaction times suitable for industrial production are sought-for.

According to the present invention there is provided a process for the reaction of preparation of compounds of formulae 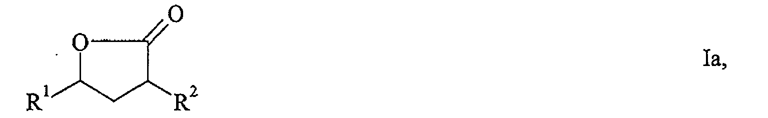 wherein R¹ is a hydrogen atom or C₁₋₁₀ alkyl, optionally substituted by one or more halogen atoms, or further substituents selected from the group consisting of C₁₋₄ alkyl, C₁₋₄ alkoxy and C₁₋₄ acyloxy, and wherein R² is selected from the group consisting of C₁₋₁₉ alkyl, aryl and aralkyl, wherein any alkyl, aryl and aralkyl substituent optionally is further substituted by one or more halogen atoms, any wherein any aryl or aralkyl residue is substituted by one or more substituents selected from the group consisting of C₁₋₄-alkoxy, C₁₋₄-acyloxy, amido, hydroxy, phenyl and *t*-butylphenyl, 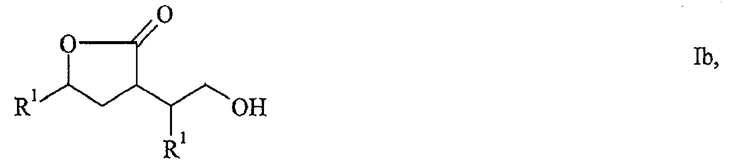 wherein R¹ is as defined above,
and/or 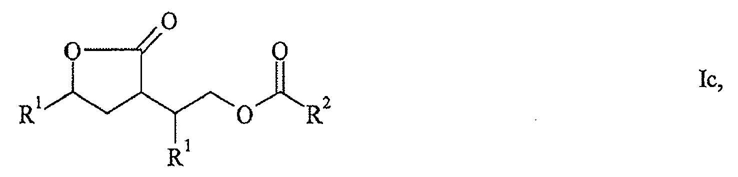 wherein R¹ and R² are as defined above,
said process comprising reacting 1 to 4 equivalents of a compound of formula 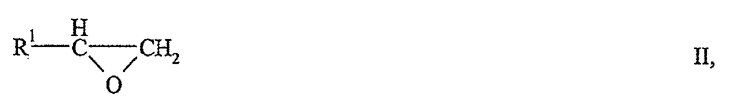 wherein R¹ is as defined above, with 1 equivalent of a compound of formula 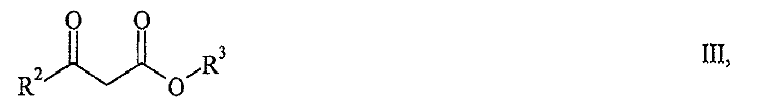 wherein R² is as defined above, and wherein R³ is selected from the group consisting of C₁₋₁₀-alkyl, aryl and aralkyl, wherein any alkyl, aryl and aralkyl optionally is substituted by one or more halogen atoms or a group consisting of C₁₋₄-alkyl or C₁₋₄-alkoxy,
characterized in that the reaction is carried out in the presence of compound of the formula

NR⁴R⁵R⁶ IV,

wherein the compound of formula IV is selected from the group consisting of
a) wherein R⁴ is hydrogen and R⁵ and R⁶ are independently selected from the group consisting of C₁₋₁₂ alkyl, optionally being further substituted with one or more halogen atoms and/or hydroxy groups,
b) wherein R⁴ is hydrogen and R⁵ and R⁶ together with the nitrogen atom form a 5 to 7-membered non-aromatic heterocyclic ring, said ring further comprising one or two nitrogen ring atoms or one oxygen ring atom,
c) wherein R⁴ is hydrogen and R⁵ and R⁶ together with the nitrogen atom form a 5 to 7-membered non-aromatic first heterocyclic ring, wherein said first ring is annellated to at least one carbocyclic or heterocyclic ring, optionally said first ring further comprising one or two nitrogen ring atoms or one oxygen ring atom,
d) wherein R⁴ is selected from the group consisting of C₁₋₁₂ alkyl, optionally being further substituted with one or more halogen atoms and/or hydroxy groups, and R⁵ and R⁶ together with the nitrogen atom form a 5 to 7-membered non-aromatic heterocyclic ring, said ring being further substituted C₁₋₁₂ alkyl, said alkyl substituent optionally being further substituted with one or more halogen atoms and/or hydroxy groups, optionally said ring further comprising one or two nitrogen ring atoms or one oxygen ring atom,
e) wherein R⁴, R⁵ and R⁶ are independently selected from the group consisting of C₁₋₁₂ alkyl, optionally being further substituted with one or more halogen atoms and/or hydroxy groups,
f) wherein R⁴ is aryl or aralkyl and R⁵ and R⁶ are independently selected from the group consisting of C₁₋₁₂ alkyl, optionally being further substituted with one or more halogen atoms and/or hydroxy groups, and
g) wherein the compound of formula IV is pyridine or a derivative thereof, and wherein the reaction optionally is carried out in the presence of a solvent and/or an additional base.

Reacting compounds of formulae II and III affords compounds of formulae Ia to Ic in different amounts. Depending on molar ratio of the compounds of formulae II and III, temperature and solvents the selectivity to obtain a certain predominant product may vary. Although the main impact of the present application is directed to the formation of acetylbutyrolactone any product of the formulae Ia to Ic obtainable by the instant process may be used as raw material for further reactions. Compounds of formula Ic can be obtained either by avoiding alkoholysis of compounds of formula Ic to compounds of formula Ib, for example by using alcohol free solvent, or by acylating compounds of formula Ib, for example by reacting with a suitable anhydrid. In the latter case the a compound according to formula Ic is obtained wherein the residue R² of the compound of formula III does not correspond to the acyl residue of said acylation reaction. Using more than one equivalents of the compound of formula II, or three equivalents in the case that an adduct of the amine with the compound of formula II is formed (as explained below in more detail), urges the reaction in the direction of forming a compound of formulae Ib and/or Ic more prodominatly then a compound of formula Ia.

Here and hereinbelow the term "alkyl" represents a linear or branched alkyl group. By using the form "C₁₋ₙ-alkyl" the alkyl group is meant having 1 to n carbon atoms. C₁₋₆-alkyl represents for example methyl, ethyl, propyl, isopropyl, butyl, isobutyl, sec-butyl, *tert*-butyl, pentyl and hexyl.

Here and hereinbelow the term "alkoxy" represents a linear or branched alkoxy group. By using the form "C₁₋ₙ-alkoxy" the alkyl group is meant having 1 to n carbon atoms. C₁₋₆-alkoxy represents for example methoxy, ethoxy, propoxy, isopropoxy, butoxy, isobutoxy, sec-butoxy, tert-butoxy, pentyloxy and hexyloxy.

Here and hereinbelow the term "alkenyl oxide" represents a linear or branched radical bearing a terminal ethylene oxide group. Examples are ethenyl oxide (EO), 1-propenyl oxide and 1-butenyl oxide.

Here and hereinbelow the term "aryl" represents an aromatic group, preferably phenyl or naphthyl.

Here and hereinbelow the term "aralkyl", represents an aromatic group having 7 or more carbon atoms, consisting of an alkyl and an aryl moiety, wherein the alkyl moiety of the aralkyl residue is a C₁₋₈ alkyl group and the aryl moiety is selected from the group consisting of phenyl, naphthyl, furanyl, thienyl, benzo[b]furanyl, benzo[b]thienyl.

A compound of formula II is selected from the group consisting of unsubstituted alkylene oxides, alkoxy ethylene oxides and alkoxy propylene oxides. Particularly preferred the compound of formula II is selected from the group consisting of ethylene oxide, propylene oxide, butylene oxide, methoxyethylene oxide, ethoxyethylene oxide, methoxy propylene oxide and ethoxy propylene oxide.

In a preferred embodiment the compound of formula II is added in an amount of 1 to 4 equivalents, more preferably of 1.0 to 2.5 equivalents.

In a preferred embodiment the compound of formula III is selected from the group consisting of alkyl, aryl and aralkyl acylacetates, preferably alkyl acetoacetates. In any case the kind of R³ groups in compounds of formula III is not important since the -OR³ group is a leaving group in the reaction. In the case an alcohol is being used as solvent preferably the alkyl moiety of the alcohol corresponds to the alkyl moiety of the leaving group -OR³ to suppress unwanted side reactions. Particularly preferred R³ is C₁₋₆-alkyl or phenyl.

The compound of the formula IV, wherein R⁴, R⁵ and R⁶ are as defined under a) to g) above, i.e. a secondary and/or tertiary amine, may be an amine where the nitrogen atom contains three substituents selected from hydrogen, alkyl and aryl or is part of at least one aromatic or non-aromatic heterocyclic ring system.
Where, according to a) above, R⁴ is hydrogen and R⁵ and R⁶ are independently selected from the group consisting of C₁₋₁₂ alkyl, optionally being further substituted with one or more halogen atoms and/or hydroxy groups, the compound of formula IV can be for example dimethylamine, diethylamine, diisopropylamine, ethylmethylamine and butylethylamine. Where, according to b) above, R⁴ is hydrogen and R⁵ and R⁶ together with the nitrogen atom form a 5 to 7-membered non-aromatic heterocyclic ring, said ring further comprising one or two nitrogen ring atoms or one oxygen ring atoms, the compound of formula IV can be for example morpholine and imidazolidine.
Where, according to c) above, R⁴ is hydrogen and R⁵ and R⁶ together with the nitrogen atom form a 5 to 7-membered non-aromatic first heterocyclic ring, wherein said first ring is annellated to at least one carbocyclic or heterocyclic ring, optionally said first ring further comprising one or two nitrogen ring atoms or one oxygen ring atom, the compound of formula IV can be for example 2-azabicyclo[2.2.1]hept-5-ene, 2,5-diazabicyclo[2.2.1]heptane and 5-methyl-2,5-diazabicyclo[2.2. 1]heptane.
Where, according to d) above, R⁴ is selected from the group consisting of C₁₋₁₂ alkyl, optionally being further substituted with one or more halogen atoms and/or hydroxy groups, and R⁵ and R⁶ together with the nitrogen atom form a 5 to 7-membered non-aromatic heterocyclic ring, said ring being further substituted C₁₋₁₂ alkyl, said alkyl substituent optionally being further substituted with one or more halogen atoms and/or hydroxy groups, optionally said ring further comprising one or two nitrogen ring atoms or one oxygen ring atom, the compound of formula IV can be for example N-methylpiperidine, *N*-ethylpiperidine, N-(2'-hydroxyethyl)piperidine, N-methylimidazolidine, N-methylimidazolidine and *N*-(2'-hydroxyethyl)imidazolidine.
Where, according to e) above, R⁴, R⁵ and R⁶ are independently selected from the group consisting of C₁₋₁₂ alkyl, optionally being further substituted with one or more halogen atoms and/or hydroxy groups, the compound of formula IV can be for example didecylmethylamine, dioctylmethylamine, ethyldiisopropylamine, 1,1,3,3-tetramethylguanidine, triethylamine, triisopropylamine, tributylamine, trimethylamine, ethyldimethylamine or methyl-di-*tert*-butylamine,
Where, according to f) above, R⁴ is aryl or aralkyl and R⁵ and R⁶ are independently selected from the group consisting of C₁₋₁₂ alkyl, optionally being further substituted with one or more halogen atoms and/or hydroxy groups, the compound of formula IV can be for example dimethylaminopyridine, diethylaminopyridine and benzyldiethylamine.
Alternatively, according to g) above, when the compound of formula IV is pyridine or a derivative thereof, wherein the derivative may carry one or more substituents independently selected from the group consisting of halogen atoms, C₁₋₁₀-alkyl and C₁₋₁₀-alkoxy, any alkyl or alkoxy optionally further substituted with one or more halogen atoms and/or hydroxy groups, the compound of formula IV can be for example N-methylpyridine, N-ethylpyridine or N-(2'-hydroxyethyl)pyridine.

Preferably the secondary and tertiary amines are selected from the group consisting of 2-azabicyclo[2.2.1]hept-5-ene, 1,4-diazabicyclo[2.2.2]octane, 1,8-diazabicyclo[5.4.0]-7-undecene (DBU), 2,5-diazabicyclo[2.2.1]heptane (DBH), didecylmethylamine (Dec₂MeN), dioctylmethylamine (Oct₂MeN), ethyldiisopropylamine (DIPEA), dimethylaminopyridine (DMAP), *N*-methyl piperidine (MePip), *N*-methyl morpholine (MeMorph), *N*-methyl imidazole (MIm), *N*-methyl imidazolidine, 1,1,3,3-tetramethylguanidine (TMG), triethylamine (TEA) and trimethylamine (TMA).

Said secondary or tertiary amine may be present in whole or parts as an alkylene oxide adduct of said amine with the compound of formula II, wherein R¹ is as defined above. The formation of such adducts is known for example from US 2173069 or US 6117948 although they have never been used in a process for the preparation of compounds of formula I.

The amine can be used alone or in combination of each other as well as in mixtures of an adduct as described above. In some cases using such an adduct in the reaction of a compound of formula II with a compound of formula III causes increased yields and/or increased selectivity compared to adding amines only. Although in most cases compound of formula II is not or only to a minor extend released from said adduct during the reaction and therefore the amount of consumed compound of formula II is increased using such adducts may have an advantageous influence on selectivity and/or yield. Especially while reacting an adduct of ethylene oxide with TEA or TMA with a compound of formula III the selectivity to one compound of formulae Ia to Ic is improved under certain reaction conditions. The tendency of the amine to form such adducts with compounds of formula II depends on the basicity of the amine. TEA and TMA easily such adducts, wherein TMG tends not to form any adducts.

In a preferred embodiment the ratio of the amine and/or the adduct to the compound of formula III is in the range from 0.0 1: 1 to 2:1 molar equivalents, more preferably from 0.2:1 to 1.0:1.

Additional bases can be selected from the group consisting of alkali metal alkoxides or hydroxides, either in solid form or as solution in a solvent.

Some secondary and tertiary amines form adducts with ethylene oxide do not react completely with the compounds of formula III and are stable under the reaction conditions.

Although in several cases remarkably amounts of the amine has to be added, it has been found that not consumed adducts can be separated and recycled. The same applies to the amines which can be easily separated from the product and recycled.

According to the invention the amine or any adduct thereof as outlined above is added as such. There is no need to add any acid or acidic salt in order to get the corresponding ammonium acid salt in the reaction mixture. In a preferred embodiment the reaction is carried out in the absence of an acid and/or halogen anions. Particularly preferred the reaction is carried out under reaction conditions to prevent formation of any ammonium halide. In a further preferred embodiment the reaction is carried out in the presence of an additional base. Such base can be selected from the group consisting of alkali or earth alkali hydroxides, carbonates and alkoxides.

In a further preferred embodiment the reaction is carried out in the presence of a complex forming compound such as borontrifluorid etherate (BF₃OEt₂) or Titan tetrakisisopropoxide (Ti(OiPr)₄ as an additive enhancing the reaction. Further possible complex forming compounds are selected from the group consisting of Group III metal halides such as AlCl₃ or transition metal halides such as FeClg.

The reaction can be performed with or without a solvent. With the proviso that the solvent does not react with one of the reagents any solvent may be used. Preferably the solvent is selected from the group consisting of water, alcohols, acetone, alkyl acetates, ethers, aromatic compounds, halogenated hydrocarbons and mixtures thereof.

If present, preferably the solvent is the corresponding alcohol of the acylacetate. In most cases the preferred alcohol is selected from the group consisting of methanol, ethanol, propanol, isopropyl alcohol, butanol, and higher alkyl alcohols, optionally in the presence of water.

Preferably, the reaction is carried out at 0 to 160 °C, particularly preferred at 20 to 120 °C, and even more particularly preferred at 40 to 120 °C.

Furthermore, the reaction is carried out at 0 to 150 bar, preferably at the pressure resulting from the vapour pressure of the reaction mixture or at a slightly higher pressure.

The reaction time is between 0.1 and 70 h, depending on the reaction temperature. In order to achieve a high selectivity in the synthesis of ABL, the reaction might be stopped before complete consumption of the alkyl acetoacetate. In the synthesis of 3-(2'-hydroxyethyl)-dihydro-2-(3H)furanone, the reaction time is preferably longer than 20 h.

Particularly preferred compounds of formula I are 3-acetyl-dihydro-2(3H)-furanone (acetylbutyrolactone = ABL) or 3-acetyl-5-methyl-dihydro-2(3H)-furanone.

The reaction can be carried out as a batch, semi batch, or continuous process. The reaction as a continuous process might be carried out in a microreactor also.

### Examples

In the Examples and in Table 1 "ep." is used as abbreviation for "equivalent". NMePip and NMeMorph are used as abbreviation for *N*-methylpiperidine and *N*-methylmorpholine, respectively.

### Example 1:

At 5 °C, a solution of methyl acetoacetate (MAA, 46.7 g, 0.40 mol) in methanol (MeOH, 144.0 g) was treated with ethylene oxide (EO, 17.5 g, 1.0 eq.) and then with 1,1,3,3-tetramethylguanidine (TMG, 46.1 g, 1.0 eq.). The reaction mixture was heated to 40 °C within 0.5 h and kept at this temperature for 4.5 h. Quantitative GC analysis indicated that 53.7% of MAA had been transformed into α-acetylbutyrolactone (ABL) with a selectivity of 75.2%.

### Example 2:

At 5 °C, a solution of MAA (46.9 g 0.40 mol) in MeOH (144.0 g) was treated with EO (25.0 g, 1.4 eq.) and then with triethylamine (TEA, 40.7 g, 1.0 eq.). The reaction mixture was heated to 60 °C within 0.5 h and kept at this temperature for 2.5 h. Quantitative GC analysis indicated that 50.8% of MAA had been transformed into ABL with a selectivity of 72.9%.

### Example 3:

At 5 °C, a solution of MAA (46.9 g 0.40 mol) in MeOH (144.0 g) was treated with EO (17.8 g, 1.0 eq.) and then with 1,8-diazabicyclo[5.4.0]-7-undecene (DBU) (62.5 g, 1.0 eq.). The reaction mixture was heated to 60°C within 0.5 h and kept at this temperature for 2.5 h. Quantitative GC analysis indicated that 59.3% of MAA had been transformed into ABL with a selectivity of 82.2%.

### Example 4:

At 5 °C, a solution of MAA (47.0 g, 0.40 mol) in MeOH (144 g) was treated with EO (35.3 g, 2.0 eq.) and then with triethylamine (TEA) (42.4 g, 1.0 eq.). The reaction mixture was heated to 60 °C within 0.5 h and kept at this temperature for 4.5 h. Quantitative GC analysis indicated that 67.7% of MAA had been transformed into ABL with a selectivity of 75.0%.

### Comparative Example 1:

At 8 °C, a solution of NaOH (16.2 g, 0.40 mol, 1.0 eq.) in MeOH (144.0 g) was treated with MAA (47.1 g, 0.40 mol) and EO (17.4 g, 0.39 mol, 0.97 eq.). The reaction mixture was heated to 60 °C within 0.5 h and kept at this temperature for 1.5 h. Quantitative GC analysis indicated that 59.5% of MAA had been transformed into ABL with a selectivity of 86.6%.

### Comparative Example 2:

At 10 °C, a solution of NaOH (16.2 g, 0.40 mol, 1.0 eq.) in MeOH (144.0 g) was treated with MAA (46.9 g, 0.40 mol) and EO (17.6 g, 0.99 eq.). The reaction mixture was heated to 25 °C within 0.5 h and kept at this temperature for 22 h. GC analysis revealed that 73.3% of MAA had been transformed into ABL with a selectivity of 71.8%.

### Example 5:

At 5 °C, a solution of MAA (46.6 g0.40 mol) in MeOH (144.0 g) was treated with EO (37.0 g, 2.1 eq.) and TMG (9.4 g, 0.2 eq.). The reaction mixture was heated to 60 °C within 0.5 h and kept at this temperature for 25 h. Qualitative GC analysis indicated that 98% of MAA had been transformed into 3-(2'-hydroxyethyl)-dihydro-2-(3H)furanone with a selectivity of >65%.

### Example 6:

At 80 °C EO (11. g, 1.0 eq.) and a mixture of ethyl acetoacetate (EAA) (32.9 g, 0.25 mol), of TEA (25.3 g, 1.0 eq.), and EtOH (61.6 g) was added in parallel within 20 min to EtOH (32.4 g) in an autoclave. The reaction mixture was stirred for an additional 2 h at 80 °C. Qualitative GC analysis indicated that 50% of EAA had been transformed into ABL with a selectivity of >60%.

### Examples 7-27:

The process of example 1, by using an EO amount, optionally a catalyst at an amount as described in Table 1, and an additive at an amount as described in Table 1, and a reaction time as described in Table 1. Turnover and selectivity as described in Table 1.

### Example 28:

Example of best mode of a series of reactions with TEA as compound of formula IV: 37 g MeOH were placed in a 250 mL autoclave, pressurized with 2-3 bar nitrogen gas and heated. After reaching 65 °C solution of MMA (39.9 g, 0.34 mol, 1 eq.) and TEA (34.4 g, 0.34 mol, 1 eq.) in methanol (27. g) and EO (30.1 g, 0.68 mol, 2 eq.) were simultaneously fed using two pumps within 11 min. After 2 h additional reaction time ABL was formed (GC analysis) with a selectivity of 77%, corresponding to 52.3% based on added MMA.

Covered ranges in the series, carried out analogously:
Temperature: 65 to 95 °C
Addition time: 9 to 13 min.
MMA added based on totally added solvent: 1.3 to 2.1 mol/L
TEA/MMA molar ratio added to the reaction mixture: 0.7 to 1.3
EO/MMA molar ratio added to the reaction mixture: 1.7 to 2.2

Results within the series of Example 28:
MMA conversion: 65.6 to 85.0%
Selectivity of ABL formation: 47.5 to 77.4%
ABL yield based on MAA: 40.4 to 52.3%

**Table 1: Examples 7-27:**

| Example | EO [eq] | Catalyst | Catalyst [eq] | Additive | Additive [eq] | t [h] | Turnover [%] | Sel. [%] |
|---|---|---|---|---|---|---|---|---|
| 7 | 1 | TMG | 1 | --- | --- | 1 | 44.7 | 76.8 |
| 8 | 1 | TMG | 1 | BF₃·OEt₂ | 0-1 | 5 | 46.3 | 66 |
| 9 | 1 | TMG | 1 | Ti(OiPr)₄ | 0.1 | 5 | 36.4 | 59.1 |
| 10 | 1 | TMG | 1 | BF₃·OEt₂ | 0.1 | 3 | 37 | 75.1 |
| 11 | 1 | TMG | 1 | Ti(OiPr)₄ | 0.1 | 3 | 42.4 | 60.6 |
| 12 | 2 | TMG | 1 | BF₃·OEt₂ | 0.1 | 5 | 82.4 | 41.9 |
| 13 | 2 | TMG | 1 | Ti(OiPr)₄ | 0.1 | 5 | 76 | 38.2 |
| 14 | 1 | Mim | 1 | --- | --- | 5 | 39.4 | 57.7 |
| 15 | 2 | Mim | 1 | --- | --- | 2 | 43.9 | 66.2 |
| 16 | 1 | DMAP | 0.2 | --- | --- | 3 | 35 | 48.9 |
| 17 | 1 | Dec₂MeN | 1 | --- | --- | 1 | 21.9 | 67.9 |
| 18 | 2 | Dec₂MeN | 1 | --- | -- | 2 | 62.9 | 71.7 |
| 19 | 1 | Oct₂MeN | 1 | --- | --- | 1 | 18 | 84.2 |
| 20 | 1 | TMA | 1 | --- | --- | 4 | 6.6 | 39 |
| 21 | 2 | TMA | 1 | --- | --- | 4 | 62.8 | 71.2 |
| 22 | 1 | TMA | 1 | --- | --- | 2 | 7 | 87.5 |
| 23 | 2 | TMA | 1 | --- | --- | 2 | 48.7 | 79.7 |
| 24 | 1 | TEA | 0.2 | --- | --- | 4 | 27.4 | 58.6 |
| 25 | 1 | Pyridin | 0.99 | --- | --- | 1 | 42.4 | 41.4 |
| 26 | 1 | NMePip | 1 | --- | --- | 4 | 18.4 | 65.1 |
| 27 | 1 | NMeMorph | 1 | --- | --- | 4 | 27.4 | 83.0 |

All reactions of examples 7 to 26 reacting 0.40 eq. MMA with the indicated amount EO have been carried out at 60 °C with 11 1 eq. MeOH as solvent. Examples 27 and 28 have been carried out with 0.10 eq. MMA with 1 eq. EO at 60 °C with 11 eq. MeOH as solvent.

### Example 29:

Example of best mode of a series of reactions with TMG as compound of formula IV: 37 g MeOH were placed in a 250 mL autoclave, pressurized with 2-3 bar nitrogen gas and heated. After reaching 45 °C solution of MMA (35.2 g, 0.30 mol, 1 eq.) and TMG (41.5 g, 0.36 mol, 1.2 eq.) in methanol (27. g) and EO (16.3 g, 0.37 mol, 1.2 eq.) were simultaneously fed using two pumps within 10 min. After 6 h additional reaction time ABL was formed (GC analysis) with a selectivity of 60.4%, corresponding to 46.0% total yield based on added MMA.

Covered ranges in the series, carried out analogously:
Temperature: 45 to 65 °C
Addition time: 9 to 11 min.
MMA added based on totally added solvent: 1.5 to 1.9 mol/L
TEA/MMA molar ratio added to the reaction mixture: 0.8 to 1.2
EO/MMA molar ratio added to the reaction mixture: 0.8 to 1.2

Results within the series of Example 29:
MMA conversion: 39.5 to 65.0%
Selectivity of ABL formation: 63.8 to 81.2%
ABL yield based on MAA: 30.8 to 46.0%

### Example 30:

Example of best mode of a series of reactions with TMA as compound of formula IV using a microreactor:
A solution A was prepared consisting of MMA (44.6 g, 0.384 mol, 1.0 eq.), TMA (16.8 g, 0.284 mol, 0.74 eq.) and MeOH (81.3 g).
30.6 g/h EO (0.695 mol/h, 1.495 eq.) and 172.8 g/h of solution A (0.465 mol/h MMA) were simultaneously fed using two pumps to a microreactor adjusted to 90 °C, said microreactor having 9.8 mL internal volume, and is acting as mixing and reaction zone. The residence time of the reaction mixture in the microreactor under the current flows was about 2.5 min. After passing through the microreactor the reaction mixture was collected in an autoclave, containing 250 mL MeOH at 60 °C and 2-3 bar under nitrogen gas. After about 1.3 h additional reaction time in the autoclave ABL was formed (GC analysis) with a selectivity of 74.8%, corresponding to a total yield of 34.8% based on added MMA, or yield of 61.9% based on added TMA. 46.6% MMA has been converted to ABL.

Covered ranges in the series, carried out analogously:
Temperature of the microreactor: 73 to 107 °C
Temperature of the autoclave: 43 to 77 °C
Dosage rate Solution A: 48.7 to 172 g/h
Dosage rate EO: 8.6 to 30.6 g/h
MMA added based solvent injected to the microreactor: 1.9 mol/L
TEA/MMA molar ratio added to the reaction mixture: 0.75
EO/MMA molar ratio added to the reaction mixture: 1.5

Results within the series of Example 30:
MMA conversion: 38.7 to 66.3%
Selectivity of ABL formation: 50.5 to 78.3%
ABL yield based on MAA: 30.3 to 39.7%

## Claims

1. A process for the preparation of compounds of formulae preparation of compounds of formulae 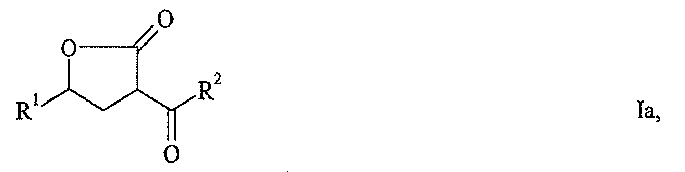 wherein R¹ is a hydrogen atom or C₁₋₁₀ alkyl, optionally substituted by one or more halogen atoms, or further substituents selected from the group consisting of C₁₋₄ alkyl, C₁₋₄ alkoxy and C₁₋₄ acyloxy, and wherein R² is selected from the group consisting of C₁₋₁₉ alkyl, aryl and aralkyl, wherein any alkyl, aryl and aralkyl substituent optionally is further substituted by one or more halogen atoms, any wherein any aryl or aralkyl residue is substituted by one or more substituents selected from the group consisting of C₁₋₄-alkoxy, C₁₋₄-acyloxy, amido, hydroxy, phenyl and *t*-butylphenyl, 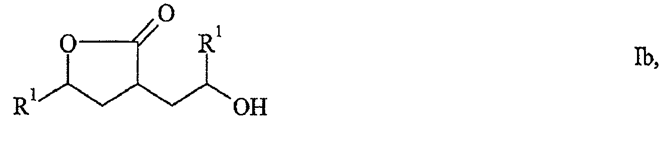 wherein R¹ is as defined above,
and/or 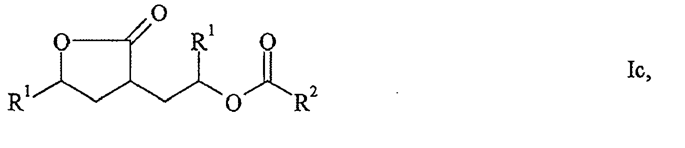 wherein R¹ and R² are as defined above,
said process comprising reacting 1 to 4 equivalents of a compound of formula 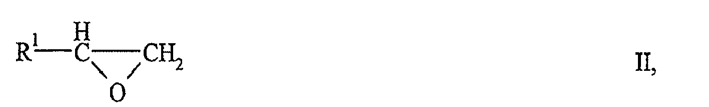 wherein R¹ is as defined above, with 1 equivalent of a compound of the formula 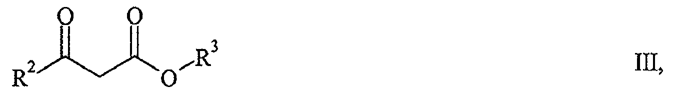 wherein R² is as defined above, and wherein R³ is selected from the group consisting of C₁₋₁₀-alkyl, aryl and aralkyl, wherein any alkyl, aryl and aralykl substituent optionally is substituted by one or more halogen atoms or a group consisting of C₁₋₄-alkyl or C₁₋₄-alkoxy, **characterized in that** the reaction is carried out in the presence of a compound of formula
NR⁴R⁵R⁶ IV,
wherein the compound of formula IV is selected from the group consisting of
a) wherein R⁴ is hydrogen and R⁵ and R⁶ are independently selected from the group consisting of C₁₋₁₂ alkyl, optionally being further substituted with one or more halogen atoms and/or hydroxy groups,
b) wherein R⁴ is hydrogen and R⁵ and R⁶ together with the nitrogen atom form a 5 to 7-membered non-aromatic heterocyclic ring, said ring further comprising one or two nitrogen ring atoms or one oxygen ring atom,
c) wherein R⁴ is hydrogen and R⁵ and R⁶ together with the nitrogen atom form a 5 to 7-membered non-aromatic first heterocyclic ring, wherein said first ring is annellated to at least one carbocyclic or heterocyclic ring, optionally said first ring further comprising one or two nitrogen ring atoms or one oxygen ring atom,
d) wherein R⁴ is selected from the group consisting of C₁₋₁₂ alkyl, optionally being further substituted with one or more halogen atoms and/or hydroxy groups, and R⁵ and R⁶ together with the nitrogen atom form a 5 to 7-membered non-aromatic heterocyclic ring, said ring being further substituted C₁₋₁₂ alkyl, said alkyl substituent optionally being further substituted with one or more halogen atoms and/or hydroxy groups, optionally said ring further comprising one or two nitrogen ring atoms or one oxygen ring atom,
e) wherein R⁴, R⁵ and R⁶ are independently selected from the group consisting of C₁₋₁₂ alkyl, optionally being further substituted with one or more halogen atoms and/or hydroxy groups,
f) wherein R⁴ is aryl or aralkyl and R⁵ and R⁶ are independently selected from the group consisting of C₁₋₁₂ alkyl, optionally being further substituted with one or more halogen atoms and/or hydroxy groups, and
g) wherein the compound of formula IV is pyridine or a derivative thereof, and wherein the reaction optionally is carried out in the presence of a solvent and/or an additional base.

2. The process of claim 1, wherein the compound of formula II is selected from the group consisting of unsubstituted alkylene oxides, alkoxy ethylene oxides and alkoxy propylene oxides.

3. The process of claims 1 or 2, wherein the compound of formula III is selected from the group consisting of alkyl, aryl and aralkyl acylacetates, preferably are alkyl acetoacetates.

4. The process of any of claims 1 to 3, wherein the secondary and tertiary amine is selected from the group consisting of 1,8-diazabicyclo[5.4.0]-7-undecene, didecylmethylamine, dimethylaminopyridine, dioctylmethylamine, ethyldiisopropylamine, *N*-methyl piperidine, *N*-methyl morpholine, *N*-methyl imidazol, *N*-methyl imidazolidine, 1,1,3,3-tetramethylguanidine, triethylamine and trimethylamine.

5. The process of any of claims 1 to 4, wherein the secondary or tertiary amine is present as an alkylene oxide adduct of said amine with the compound of formula II, wherein R¹ is as defined in claim 1.

6. The process of any of claims 1 to 5, wherein the ratio of the amine and/or adduct to the compound of formula III is in the range from 0.01:1 to 2.0:1 molar equivalents.

7. The process of any of claims 1 to 6, wherein the reaction is carried out in the absence of an acid and/or halogen anions.

8. The process of any of claims 1 to 7, wherein the reaction is carried out at a temperature from 0 to 160 °C.

9. The process of any of claims 1 to 8, wherein the reaction is carried out at a pressure from 1 to 150 bar.

10. The process of any of claims 1 to 9, wherein the reaction time is between 0.1 and 70 h.

11. The process of any of claims 1 to 10, wherein the compound of formula I is 3 -acetyl-dihydro-2(3H)-furanone or 3-acetyl-5-methyl-dihydro-2(3H)-furanone.
